# FASCICULE DE BREVET EUROPEEN

(11) **EP 1 064 249 B1**
(45) Date de publication et mention de la délivrance du brevet: **05.11.2003**
(21) Numéro de dépôt: 98959953.5
(22) Date de dépôt: 10.12.1998
(51) Int. Cl.: C07C 67/62, C07C 69/54, C07C 67/03, C07C 67/08

(54) **PROCEDE DE CONDITIONNEMENT D'ACRYLATES D'ALKYLE A LONGUE CHAINE**
VERFAHREN ZUM BEWAHREN VON LANGKETTIGEN ALKYLACRYLATEN
METHOD FOR CONDITIONING LONG CHAIN ALKYL ACRYLATES

(30) Priorité: 15.12.1997 FR 9715874
(43) Date de publication de la demande: 03.01.2001
(73) Titulaire: Elf Atochem S.A., 92800 Puteaux Hauts-de-Seine (FR)
(72) Inventeur: PAUL, Jean-Michel, F-57070 Metz (FR); GAMET, Jean-Paul, F-62690 Savy Berlette (FR)
(74) Mandataire: Rieux, Michel
(86) Numéro de dépôt international: FR9802690
(87) Numéro de publication internationale: WO99031042

(56) Documents cités:
- EP-A- 0 013 836
- FR-A- 2 224 437
- FR-A- 2 602 229

## Description

La présente invention porte sur un procédé de conditionnement d'acrylates d'alkyle à longue chaîne.

Les acrylates d'alkyle à longue chaîne, c'est-à-dire dont le nombre d'atomes de carbone est d'au moins 18, sont utilisés comme comonomères dans des formulations destinées à l'abaissement du point de figeage des huiles paraffiniques. Les huiles brutes peuvent contenir des paraffines dont la nature et la teneur sont directement liées au site d'extraction. A une température qui dépend de la nature des huiles, il y a cristallisation de ces paraffines, ce qui entraîne une diminution de leur fluidité avec, pour conséquence, une gêne dans leur transport et leur stockage. Une solution intéressante consiste à abaisser le point de figeage de ces huiles par ajout d'un additif. Dans le brevet français FR-A-1 575 984, a été proposée l'utilisation de composés macromoléculaires de type peignes construits sur le modèle d'une chaîne hydrocarbonée portant des chaînes latérales assez longues (C₁₄ à C₃₀). D'autres brevets, tels que le brevet français FR-B-2 128 589 et le brevet américain US-A-2 839 512, revendiquent l'utilisation de copolymères d'acrylates en C₁₈-C₃₀, de préférence en C₁₈-C₂₂, et d'un monomère hétérocyclique de type vinylpyridine. La demande internationale WO 97/34940 revendique, pour sa part, l'utilisation de copolymères acryliques préparés à partir d'acrylates linéaires en C₂₄ à C₆₀ (de préférence en C₃₀ à C₄₀) et d'acrylates en C₁₀ à C₂₂ (acrylate de béhényle par exemple).

Le problème que l'invention vise à résoudre est le suivant :

Les acrylates d'alkyle à longue chaîne (>C₁₈), utiles dans le contexte qui vient d'être indiqué, sont d'une manipulation difficile du fait que ce sont des solides à haut point de fusion. Leur manipulation impose de les avoir et de les maintenir à l'état liquide (au-dessus de leur température de fusion). Leur stockage doit être fait dans des conteneurs réchauffés ou dans des fûts ou tonnelets réchauffables.
- lorsqu'ils sont stockés dans des conteneurs réchauffés, il faut, du fait de leur température de fusion élevée, les maintenir à haute température, ce qui est contraignant et dangereux (risques de polymérisation) ;
- lorsqu'ils sont conditionnés en fûts, de longues et fastidieuses opérations de fusion à haute température dans des enceintes chauffées sont nécessaires avant utilisation avec les risques de polymérisation que cela comporte.

La Société déposante a recherché une solution pour faciliter la manipulation des acrylates d'alkyle à longue chaîne tout en diminuant les risques de polymérisation, et elle propose, conformément à la présente invention, un procédé de conditionnement conduisant à un produit constitué par un mélange dudit acrylate et d'un solvant, lequel mélange a une température de fusion inférieure, et notamment nettement inférieure, à celle dudit acrylate. La manipulation de ce dernier en est d'autant facilitée et les risques de polymérisation lors de la fonte du produit notamment réduits ; sous cette présentation, ledit acrylate peut être conditionné sous forme liquide à basse température dans des conteneurs réchauffés sans risque de polymérisation ; il peut être également stocké en fûts et fondu lors de son utilisation ultérieure en toute sécurité.

Par ailleurs, ledit solvant peut déjà, de façon très avantageuse, être introduit dans le réacteur de synthèse dudit acrylate, un autre avantage de l'invention consistant alors en une plus grande facilité de vidange du réacteur du brut de réaction. Ainsi, en abaissant le point de fusion, on évite d'autant les risques de cristallisation du produit lors de la vidange du réacteur dans les conduites de transfert vers les conteneurs ou les fûts.

La présente invention a donc pour objet un procédé de conditionnement d'un acrylate d'alkyle à longue chaîne de formule générale : 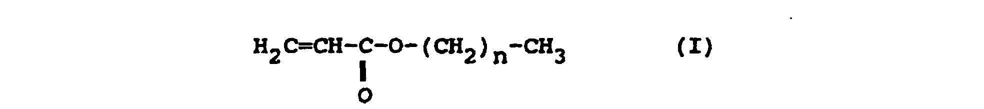 dans laquelle n est compris entre 18 et 60,
ledit acrylate pouvant également se présenter sous la forme d'un mélange d'acrylates (I) ayant des longueurs de chaîne comprises entre 18 et 60 atomes de carbone, caractérisé par le fait qu'on forme un mélange dudit acrylate (I) et d'un solvant (SL) dont la nature et la quantité sont choisies pour que ledit mélange constitue un produit dont le point de fusion est inférieur à celui dudit acrylate (I).

Conformément à un mode de réalisation préféré du procédé selon la présente invention, on introduit le solvant (SL) dans le réacteur de fabrication de l'acrylate (I) lors du chargement des réactifs et/ou pendant la réaction et/ou après la fin de la réaction, ledit solvant (SL) étant choisi pour être inerte vis-à-vis du milieu réactionnel dans lequel il est introduit. On peut ainsi choisir le solvant (SL) parmi les hydrocarbures aliphatiques ou aromatiques, tels que les fractions d'hydrocarbures pétroliers. A titre d'exemples particuliers de solvants (SL), on peut citer les xylènes, les éthyl benzènes et les triméthylbenzènes.

On introduit avantageusement le solvant (SL) dans une quantité telle qu'il représente de 10 à 70% en poids, en particulier de 40 à 70% en poids, par rapport à l'acrylate (I).
- Dans le cas d'un procédé selon la présente invention associé à la fabrication de l'acrylate (I) par transestérification entre un ester léger, liquide à température ambiante, de formule (II) : 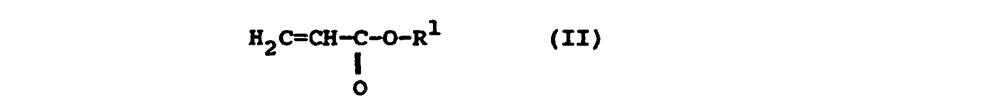
dans laquelle R¹ représente alkyle en C₁-C₄
et un alcool lourd, solide à la température ambiante, de formule (III) :

CH₃(CH₂)ₙOH (III)

dans laquelle n vaut 18 à 60,
en présence d'au moins un catalyseur de transestérification et d'au moins un inhibiteur de polymérisation, sous bullage d'air ou d'air appauvri, l'alcool léger R¹OH formé distillant sous la forme d'un azéotrope ester léger (II)/R¹OH, et l'ester léger (II) en excès étant éliminé par distillation en fin de réaction,
on peut ajouter le solvant (SL) à la charge initiale et/ou après l'élimination de l'ester léger (II), ledit solvant (SL) possédant une température d'ébullition supérieure à celle de l'ester léger (II) lorsqu'il est introduit en totalité ou en partie avec la charge initiale.

La transestérification est par ailleurs réalisée dans les conditions classiques, c'est-à-dire en excès molaire d'ester acrylique léger (II), le rapport molaire ester (II)/alcool (III) étant généralement compris entre 1,1 et 6 et plus particulièrement entre 1,1 et 3,5, un rapport molaire élevé étant favorable à la conversion de l'alcool(III) mais pénalisant sur le plan de la productivité. La température de transestérification est généralement comprise entre 80 et 160°C.

Comme indiqué ci-dessus, la transestérification s'effectue en règle générale en présence d'un catalyseur tel que le dibutylétain et les autres produits de cette famille incluant par exemple, les distannoxanes, les titanates, tels que, par exemple, le titanate d'isopropyle, les alcoolates de sodium de calcium et de magnésium, les acétyl-acétonates de zirconium et de calcium et autres produits de cette famille. La quantité de catalyseur mise en oeuvre est comprise entre 0,001 et 0,05 mole pour 100 moles d'alcool (III), et, de préférence, entre 0,001 et 0,01 mole pour 100 moles d'alcool (III).

Comme indiqué ci-dessus également, la transestérification s'effectue en règle générale en présence d'au moins un inhibiteur de polymérisation choisi parmi les produits suivants bien connus de l'homme du métier : phénothiazine, hydroquinone, ester méthylique de l'hydroquinone, ditert.-butyl catéchol, à raison notamment de 0,05 à 0,5% en masse, calculé sur la base de l'alcool (III).
- Dans le cas d'un procédé selon la présente invention associé à la fabrication de l'acrylate (I) par estérification entre l'acide acrylique et l'alcool lourd, solide à la température ambiante, de formule (III) :

   CH₃(CH₂)ₙOH (III)

   dans laquelle n vaut 18 à 60
   en présence d'au moins un catalyseur d'estérification et d'au moins un inhibiteur de polymérisation et dans un solvant léger (Sl) susceptible de former un azéotrope avec l'eau, l'eau de réaction distillant sous forme d'un azéotrope solvant léger (Sl)/eau, on peut ajouter le solvant (SL) à la charge initiale et/ou en fin de réaction d'estérification, ledit solvant (SL) possédant une température d'ébullition supérieure à celle du solvant léger (Sl) lorsqu'il est introduit en totalité ou en partie avec la charge initiale.

L'estérification est par ailleurs réalisée dans les conditions classiques, le rapport molaire alcool (III)/acide acrylique étant généralement compris entre 1,05 et 5, et la température étant comprise entre 80 et 160°C. Le solvant léger (Sl) facilite l'élimination de l'eau en formant un azéotrope avec elle, déplaçant l'équilibre d'estérification dans le sens de formation de l'acrylate (I). Il peut être choisi parmi les solvants aliphatiques et aromatiques qui forment un azéotrope avec l'eau, par exemple l'hexane, le cyclohexane et le toluène.

Comme indiqué ci-dessus, l'estérification s'effectue en règle générale en présence d'un catalyseur choisi par exemple parmi les acides minéraux forts tels que H₂SO₄, l'acide méthane sulfonique, et les résines échangeuses d'ions cationiques sous forme H⁺, par exemple les résines cationiques obtenues en sulfonant un copolymère de styrène et de divinylbenzène, ayant des teneurs en H⁺ comprises entre 0,05 et 0,2 équivalent/kg de réactifs.

De même, l'estérification est généralement conduite en présence d'un inhibiteur de polymérisation, tel que ceux indiqués ci-dessus pour la transestérification, à des teneurs identiques.

Conformément à une autre variante du procédé selon la présente invention, le solvant (SL) est ajouté en fin de réaction, avantageusement alors que le produit de réaction est à une température de 60 à 90°C.

Le procédé selon l'invention, mis en oeuvre dans la réaction de transestérification ou d'estérification, conduit donc avantageusement à un mélange liquide homogène à chaud dans ledit réacteur, ledit mélange pouvant alors être facilement vidangé par gravité en conteneurs chauffés ou en fûts. En abaissant ainsi le point de fusion de l'acrylate (I) par son mélange avec le solvant (SL), on évite de surchauffer les conduites de transfert vers les conteneurs ou fûts et les risques de polymérisation à la paroi. Par rapport à la technique antérieure :
- les conteneurs peuvent être maintenus à une température plus basse, ce qui réduit les risques de polymérisation au stockage ;
- si le mélange ester (I)/solvant (SL) a été conditionné en fûts, le réchauffage de ces derniers en vue de faire fondre le produit pour une utilisation ultérieure est facilité et les risques de polymérisation minimisés.

La présente invention conduit avantageusement à un mélange ester (I)/solvant (SL) présentant une température de fusion inférieure d'au moins 20°C à celle de l'acrylate (I).

Les Exemples suivants illustrent la présente invention sans toutefois en limiter la portée.

### Exemple 1

Dans un réacteur agité mécaniquement, chauffé à l'aide d'un chauffe-réacteur électrique surmonté d'une colonne à distiller, on charge :
- 300 g d'un alcool lourd : alcool linéaire en C₁₈-C₄₀, dont la répartition est centrée sur la fraction C₂₈-C₃₂, présentant une teneur en alcools de 85% (les 15% restants étant des hydrocarbures linéaires saturés), de masse molaire moyenne mesurée par indice OH de 460 ;
- 138,5 g d'acrylate d'éthyle (rapport molaire acrylate d'éthyle/alcool ci-dessus = 2,5) ;
- 327 g de 1,2,4-triméthylbenzène (SOLVESSO™ 150) ;
- 0,17 g d'éther monométhylique de l'hydroquinone.

On effectue un séchage du milieu réactionnel en distillant l'eau sous forme d'un azéotrope acrylate d'éthyle/eau, puis on ajoute 0,82 g d'acétylacétonate de zirconium.

La réaction est effectuée sous pression réduite (3,99 x 10⁴ - 2,66 x 10⁴ Pa (300-200 mmHg), à une température comprise entre 100 et 120°C. L'alcool léger (éthanol) est distillé sous forme d'un azéotrope acrylate d'éthyle/éthanol, afin de déplacer l'équilibre de transestérification dans le sens de formation de l'acrylate lourd.

Lorsque la réaction est terminée, on distille l'acrylate d'éthyle en excès et on ajoute un complément de 169 g de 1,2,4-triméthyl benzène, afin d'avoir dans le mélange final le pourcentage massique souhaité de 60%.

Le brut final liquide et homogène à chaud dans le réacteur (808 g) est vidangé vers 70°C.

Après refroidissement à température ambiante, le produit final se présente sous la forme d'un solide qui fond vers 48-50°C (contre 82-83°C pour l'acrylate lourd).

Ce produit peut être stocké à 48°C ou à température ambiante (avec dans ce cas nécessité de la refondre avant utilisation), sans risque de polymérisation.

### EXEMPLE 2

On reprend l'Exemple 1 en chargeant la totalité du 1,2,4-triméthylbenzène en fin d'opération (après distillation suivie d'un strippage de l'acrylate d'éthyle).

La réaction est effectuée comme décrit à l'Exemple 1 sous pression réduite (3,99 x 10⁴ - 1,33 x 10⁴ Pa (300 à 100 mmHg)), à une température comprise entre 100 et 120°C.

L'acrylate d'éthyle résiduel est éliminé par distillation sous 1,33 x 10⁴ à 6,6 x 10³ Pa (100 à 50 mm Hg).

Un strippage supplémentaire est ensuite effectué sous une pression de 2,6 x 10³ Pa (20 mmHg) à l'aide d'un bullage d'air afin de chasser les traces d'acrylate d'éthyle résiduel (< 200 ppm dans le produit final).

496 g de 1,2,4-triméthylbenzène sont alors introduits dans le réacteur sous agitation à 70-80°C, en 20 minutes.

Le mélange final est vidangé vers 70°C.

On obtient ainsi 809 g d'un mélange acrylate lourd/1,2,4-triméthylbenzène présentant les mêmes caractéristiques qu'à l'Exemple 1.

### EXEMPLE 3

On reprend l'Exemple 2 en remplaçant le 1,2,4-triméthylbenzène par du xylène dans les proportions massiques acrylate lourd/xylène = 50/50.

Le point de fusion du mélange final est de 59-60°C (contre 82-83°C pour l'acrylate lourd).

### EXEMPLE 4

Dans l'appareillage décrit à l'Exemple 1, modifié par ajout d'un décanteur en tête de colonne, on introduit :
- 300 g de l'alcool lourd utilisé à l'Exemple 1 ;
- 41,9 g d'acide acrylique ;
- 150 g de toluène ;
- 3,5 g d'acide sulfurique compté en 100% ;
- 0,17 g d'éther monométhylique de l'hydroquinone/0,17 g d'hydroquinone.

Pendant toute la durée de l'opération, on maintient un léger bullage d'air dans le réacteur.

La réaction est effectuée sous pression réduite, à une température comprise entre 90 et 115°C.

L'eau de réaction est distillée sous forme d'un azéotrope toluène/eau, afin de déplacer l'équilibre d'estérification dans le sens de la formation de l'acrylate lourd. Le toluène décanté est recyclé en permanence dans le réacteur.

En fin de réaction, le toluène est distillé et l'excès d'acide acrylique et le catalyseur sont neutralisés à l'aide de Ca(OH)₂.

493 g de 1,2,4-triméthylbenzène (60% en masse par rapport au produit final) sont introduits dans le réacteur vers 85°C en 20 minutes.

Le brut est vidangé à chaud sous forme liquide au travers d'un filtre chauffant.

Le mélange final se présente sous la forme d'un solide ayant un point du fusion d'environ 50°C.

## Revendications

1. Procédé de conditionnement d'un acrylate d'alkyle à longue chaîne de formule générale (I) : 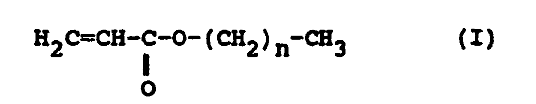 dans laquelle n est compris entre 18 et 60,
ledit acrylate pouvant également se présenter sous la forme d'un mélange d'acrylates (I) ayant des longueurs de chaîne comprises entre 18 et 60 atomes de carbone,
**caractérisé par le fait qu'**on forme un mélange dudit acrylate (I) et d'un solvant (SL) dont la nature et la quantité sont choisis pour que ledit mélange constitue un produit dont le point de fusion est inférieur à celui dudit acrylate (I).

2. Procédé de conditionnement selon la revendication 1, **caractérisé par le fait que** l'on introduit le solvant (SL) dans le réacteur de fabrication de l'acrylate (I) lors du chargement des réactifs et/ou pendant la réaction et/ou après la fin de la réaction, ledit solvant (SL) étant choisi pour être inerte vis-à-vis du milieu réactionnel dans lequel il est introduit.

3. Procédé selon l'une des revendications 1 et 2, **caractérisé par le fait que** l'on choisit le solvant (SL) parmi les hydrocarbures aliphatiques ou aromatiques, tels que les fractions d'hydrocarbures pétroliers.

4. Procédé selon la revendication 3, **caractérisé par le fait que** l'on choisit le solvant (SL) parmi les xylènes, les éthyl benzènes et les triméthylbenzènes.

5. Procédé selon l'une des revendications 1 à 4, **caractérisé par le fait que** l'on introduit le solvant (SL) dans une quantité telle qu'il représente de 10 à 70% en poids, en particulier de 40 à 70% en poids, par rapport à l'acrylate (I).

6. Procédé selon l'une des revendications 2 à 5, associé à la fabrication de l'acrylate (I) par transestérification entre un ester léger, liquide à température ambiante, de formule (II) : 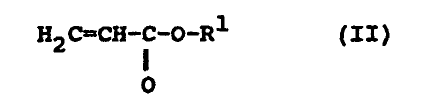 dans laquelle R¹ représente alkyle en C₁-C₄
et un alcool lourd, solide à la température ambiante, de formule (III) :
CH₃(CH₂)ₙOH (III)
dans laquelle n vaut 18 à 60,
en présence d'au moins un catalyseur de transestérification et d'au moins un inhibiteur de polymérisation, sous bullage d'air ou d'air appauvri, l'alcool léger R¹OH formé distillant sous la forme d'un azéotrope ester léger (II)/R¹OH, et l'ester léger (II) en excès étant éliminé par distillation en fin de réaction,
**caractérisé par le fait que** l'on ajoute le solvant (SL) à la charge initiale et/ou après l'élimination de l'ester léger (II), ledit solvant (SL) possédant une température d'ébullition supérieure à celle de l'ester léger (II) lorsqu'il est introduit en totalité ou en partie avec la charge initiale.

7. Procédé selon l'une des revendications 2 à 5, associé à la fabrication de l'acrylate (I) par estérification entre l'acide acrylique et l'alcool lourd, solide à la température ambiante, de formule (III) :
CH₃(CH₂)ₙOH (III)
dans laquelle n vaut 18 à 60
en présence d'au moins un catalyseur d'estérification et d'au moins un inhibiteur de polymérisation et dans un solvant léger (Sl) susceptible de former un azéotrope avec l'eau, l'eau de réaction distillant sous forme d'un azéotrope solvant léger (Sl)/eau,
**caractérisé par le fait que** l'on ajoute le solvant (SL) à la charge initiale et/ou en fin de réaction d'estérification, ledit solvant (SL) possédant une température d'ébullition supérieure à celle du solvant léger (Sl) lorsqu'il est introduit en totalité ou en partie avec la charge initiale.

8. Procédé selon l'une des revendications 6 et 7, **caractérisé par le fait que** le solvant (SL) est ajouté en fin de réaction alors que le produit de réaction est à une température de 60 à 90°C.

9. Procédé selon l'une des revendications 2 à 8, **caractérisé par le fait qu'**il conduit à un mélange liquide homogène à chaud dans le réacteur, ledit mélange étant alors vidangé par gravité en conteneurs chauffés ou en fûts.

10. Procédé selon l'une des revendications 1 à 9, **caractérisé par le fait qu'**il conduit à un mélange ester (I)/solvant (SL) présentant une température de fusion inférieure d'au moins 20°C à celle de l'acrylate (I).

## Patentansprüche

1. Verfahren zur Konfektionierung eines langkettigen Alkylacrylat der folgenden allgemeinen Formel (I): 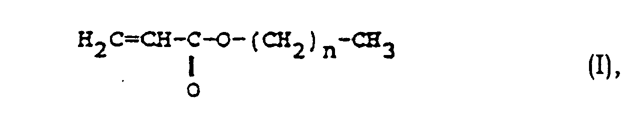 worin n im Bereich von 18 bis 60 liegt,
wobei das Acrylat auch in Form eines Gemisches von Acrylaten
(I) vorliegen kann, deren Kettenlänge im Bereich von 18 bis 60 Kohlenstoffatomen liegt,
**dadurch gekennzeichnet, dass** ein Gemisch des Acrylat (I) und eines Lösungsmittels (SL) gebildet wird, dessen Art und Menge so ausgewählt ist, dass das Gemisch ein Produkt ist, dessen Schmelzpunkt unter dem Schmelzpunkt des Acrylat (I) liegt.

2. Verfahren zur Konfektionierung nach Anspruch 1, **dadurch gekennzeichnet, dass** das Lösungsmittel (SL) beim Einfüllen der Reaktanten und/oder während der Umsetzung und/oder nach erfolgter Umsetzung in den Reaktor für die Herstellung des Acrylat (I) gegeben wird, wobei das Lösungsmittel (SL) so gewählt wird, dass es gegenüber dem Reaktionsmedium, in das es gegeben wird, inert ist.

3. Verfahren nach einem der Ansprüche 1 und 2, **dadurch gekennzeichnet, dass** das Lösungsmittel (SL) unter den aliphatischen oder aromatischen Kohlenwasserstoffen ausgewählt ist, beispielsweise Kohlenwasserstofffraktionen aus Erdöl.

4. Verfahren nach Anspruch 3, **dadurch gekennzeichnet, dass** das Lösungsmittel (SL) unter den Xylolen, Ethylbenzolen und Trimethylbenzolen ausgewählt ist.

5. Verfahren nach einem der Ansprüche 1 bis 4, **dadurch gekennzeichnet, dass** das Lösungsmittel (SL) in einer solchen Menge zugegeben wird, dass es 10 bis 70 Gew.-% und insbesondere 40 bis 70 Gew.-%, bezogen auf das Acrylat (I), ausmacht.

6. Verfahren nach einem der Ansprüche 2 bis 5 in Kombination mit der Herstellung des Acrylat (I) durch Umesterung eines bei Raumtemperatur flüssigen, leichten Esters der folgenden Formel (II): 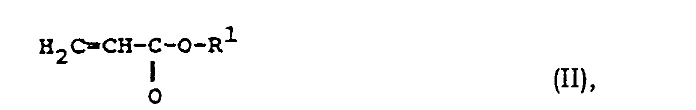 worin R¹ C₁₋₄- Alkyl bedeutet, und eines schweren, bei Raumtemperatur festen Alkohols der Formel (III):
CH₃(CH₂)ₙOH (III),
worin n 18 bis 60 bedeutet,
in Gegenwart mindestens eines Katalysators für die Umesterung und mindestens eines Polymerisationsinhibitors unter Spülen mit Luft oder abgereicherter Luft, wobei der gebildete leichte Alkohol R¹OH als Azeotrop leichter Ester (II)/R¹OH abdestilliert wird und der überschüssige leichte Ester (II) am Ende der Umsetzung durch Destillation entfernt wird, **dadurch gekennzeichnet, dass** das Lösungsmittel (SL) in die anfängliche Charge und/oder nach Beseitigung des leichten Esters (II) zugegeben wird, wobei das Lösungsmittel (SL) eine Siedetemperatur besitzt, die über der Siedetemperatur des leichten Esters (II) liegt, wenn es ganz oder teilweise in die ursprüngliche Charge gegeben wird.

7. Verfahren nach einem der Ansprüche 2 bis 5 in Kombination mit der Herstellung des Acrylat (I) durch Veresterung von Acrylsäure mit einem schweren, bei Raumtemperatur festen Alkohol der Formel (III):
CH₃(CH₂)ₙOH (III),
worin n 18 bis 60 bedeutet,
in Gegenwart mindestens eines Katalysators für die Veresterung und mindestens eines Polymerisationsinhibitors in einem leichten Lösungsmittel (Sl), das befähigt ist, mit Wasser ein Azeotrop zu bilden, wobei das bei der Umsetzung gebildete Wasser als Azeotrop leichtes Lösungsmittel (Sl)/Wasser abdestilliert wird,
**dadurch gekennzeichnet, dass** das Lösungsmittel (SL) in die anfänglich vorliegende Charge und/oder am Ende der Veresterung zugegeben wird, wobei das Lösungsmittel (SL) eine Siedetemperatur über der Siedetemperatur des leichten Lösungsmittels (Sl) besitzt, wenn es ganz oder teilweise in die anfänglich vorliegende Charge gegeben wird.

8. Verfahren nach einem der Ansprüche 6 und 7, **dadurch gekennzeichnet, dass** das Lösungsmittel (SL) am Ende der Umsetzung zugegeben wird, wenn das Reaktionsprodukt eine Temperatur von 60 bis 90 °C aufweist.

9. Verfahren nach einem der Ansprüche 2 bis 8, **dadurch gekennzeichnet, dass** es zu einem flüssigen, in der Wärme homogenen Gemisch in dem Reaktor führt, wobei das Gemisch dann mithilfe von Schwerkraft entnommen und in beheizte Behälter oder Fässer überführt wird.

10. Verfahren nach einem der Ansprüche 1 bis 9, **dadurch gekennzeichnet, dass** es zu einem Gemisch Ester (I)/Lösungsmittel (SL) führt, das eine Schmelztemperatur aufweist, die mindestens 20 °C unter der Schmelztemperatur des Acrylat (I) liegt.

## Claims

1. Process for packaging a long-chain alkyl acrylate of general formula (I) : 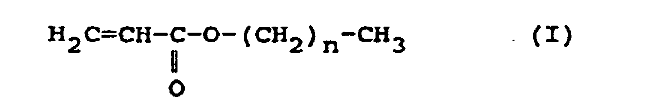 in which n is between 18 and 60,
it being possible for the said acrylate also to be in the form of a mixture of acrylates (I) having chain lengths of between 18 and 60 carbon atoms, **characterized in that** a mixture of the said acrylate (I) and a solvent (SL) is formed whose nature and amount are chosen such that the said mixture constitutes a product whose melting point is less than that of the said acrylate (I).

2. Packaging process according to Claim 1, **characterized in that** the solvent (SL) is introduced into the reactor for manufacturing the acrylate (I) during the loading of the reagents and/or during the reaction and/or after the end of the reaction, the said solvent (SL) being chosen such that it is inert with respect to the reaction medium into which it is introduced.

3. Process according to either of Claims 1 and 2, **characterized in that** the solvent (SL) is chosen from aliphatic and aromatic hydrocarbons, such as petroleum hydrocarbon fractions.

4. Process according to Claim 3, **characterized in that** the solvent (SL) is chosen from xylenes, ethylbenzenes and trimethylbenzenes.

5. Process according to one of Claims 1 to 4, **characterized in that** the solvent (SL) is introduced in an amount such that it represents from 10 to 70% by weight, in particular from 40 to 70% by weight, relative to the acrylate (I).

6. Process according to one of Claims 2 to 5, combined with the manufacture of the acrylate (I) by transesterification between a light ester, which is liquid at room temperature, of formula (II) : 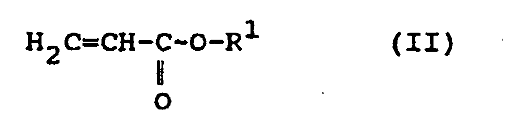 in which R¹ represents C₁-C₄ alkyl
and a heavy alcohol, which is solid at room temperature, of formula (III):
CH₃(CH₂)ₙOH (III)
in which n is from 18 to 60,
in the presence of at least one transesterification catalyst and at least one polymerization inhibitor, while bubbling with air or depleted air, the light alcohol R¹OH formed distilling off in the form of an azeotrope of light ester (II)/R¹OH, and the excess light ester (II) being removed by distillation at the end of the reaction,
**characterized in that** the solvent (SL) is added to the initial load and/or after removal of the light ester (II), the said solvent (SL) having a boiling point which is greater than that of the light ester (II) when it is introduced in total or in part with the initial load.

7. Process according to one of Claims 2 to 5, combined with the manufacture of the acrylate (I) by esterification between the acrylic acid and the heavy alcohol, which is solid at room temperature, of formula (III):
CH₃(CH₂)ₙOH (III)
in which n is from 18 to 60,
in the presence of at least one esterification catalyst and of at least one polymerization inhibitor and in a light solvent (Sl) which is capable of forming an azeotrope with water, the reaction water distilling off in the form of an azeotrope of light solvent (Sl)/water,
**characterized in that** the solvent (SL) is added to the initial load and/or at the end of the esterification reaction, the said solvent (SL) having a boiling point which is higher than that of the light solvent (Sl) when it is introduced in total or in part with the initial load.

8. Process according to either of Claims 6 and 7, **characterized in that** the solvent (SL) is added at the end of the reaction while the reaction product is at a temperature of from 60 to 90°C.

9. Process according to one of Claims 2 to 8, **characterized in that** it leads to a liquid mixture which is homogeneous when hot in the reactor, the said mixture then being emptied out by gravity into heated containers or into drums.

10. Process according to one of Claims 1 to 9, **characterized in that** it leads to an ester (I)/solvent (SL) mixture which has a melting point that is at least 20°C lower than that of the acrylate (I).
